# EUROPEAN PATENT APPLICATION

(11) **EP 3 761 320 A1**
(43) Date of publication of application: **06.01.2021**
(21) Application number: 19184222.8
(22) Date of filing: 03.07.2019
(51) Int. Cl.: G21F 3/00, A61B 6/10, A61N 5/10

(54) **RADIATION SHIELD FOR OPERATING TABLE**

(71) Applicant: Koninklijke Philips N.V., 5656 AG Eindhoven (NL)
(72) Inventor: VAN DE GEER, Cedric, 5656 AE Eindhoven (NL)
(74) Representative: de Haan, Poul Erik

(57) **Abstract**

A shielding system of an operating table is provided for shielding a body from scatter radiation during a medical intervention. The shielding system comprises a frame attachable to an operating table, and a shield comprising material for blocking radiation, the shield being configured for being mounted on the frame, wherein the shielding system is adapted to be collapsed by displacement of at least part of the shield or parts thereof in a vertical direction. An operating table including such shielding system is also provided.

## Description

### FIELD OF THE INVENTION

The invention relates to the field of radiation shielding. More specifically, it relates to shielding and protection of medical personnel against radiation which is damaging to living matter, such as X-radiation, particle bombardment, scatter radiation and similar, during an intervention.

### BACKGROUND OF THE INVENTION

Hybrid Operating Rooms (OR) are operating rooms which combine the traditional surgical theater with highly advanced imaging techniques such as CT scanners, MRI scanners, and similar. Hybrid OR are used in many interventions, such as minimally invasive surgeries like for instance Transcatheter Aortic Valve Implantation (TAVI) or Endovascular Aneurysm Repair (EVAR).

Such Hybrid Operating Rooms can be found in hospitals, clinics, medical research facilities in universities, etc. They also combine multidisciplinary personnel, including surgeons, nurses, anesthesiologists, information technology staff, imaging specialists, etc. During an intervention, imaging equipment may be used, including X-rays and/or other highly energetic and/or ionizing radiation, which poses a health risk to the personnel of the operating room. As radiation exposure is cumulative, working in these environments may result in a large cumulative radiation exposure for the staff.

Hybrid ORs can be configured and adapted in order to minimize exposure of radiation, e.g. scatter radiation, to the medical staff. Personnel usually receive high dose of scatter radiation if standing on the side of the operating table (OT) where the radiograph tube for horizontal beam is placed. This position should be avoided. As an example, in some interventions, the anesthesiologist can be positioned at the head of the table, which results in lower exposure, as explained in "Anesthesiologists in the Neurointerventional Suite - What Is Appropriate Radiation Protection?", editorial views, Anesthesiology, V 114, No 3, March 2011.

However, some medical personnel has higher risk of exposure or accidental exposure than others, as safety guidelines do not apply in the same way to all medical personnel. For example, some safety guidelines that apply to staff of a hybrid OR, like eye protection requirements for the main surgeon or interventional radiologist, do not apply to the anaesthesiologist, as explained in "Radiation exposure to anaesthetists during endovascular procedures", Anaesthesia. 2015;70(1):47-50. This might lead to dangerous exposure, especially in situations of high radiation dose like during an emergency, where specifically the anaesthesiologist has to be close to the patient. Generally there is low risk awareness among supporting personnel in the hybrid operating room. Even though some modern hospitals position the anaesthesiologist in the low-dose technical room, most other hospitals do not have this feature or possibility. The combination of these factors can lead to unnecessary and dangerous exposure to scatter radiation, especially in the long term.

The several shielding products that do exist in countering the effects of radiation, create a barrier between the patient and the hybrid OR staff and are often ill-used or discarded altogether. Examples of these are mobile, wheeled shields or operation-table mounted shielding. Both limit patient access. The mobile shield will often be moved out of the way when the C-arm position changes, and consequently lose its functionality. Fixed shieldings are often discarded, as they are unwieldy, and frequently unable to adapt to changing circumstances, like complications during the procedure, change of C-arm position, and consequently, change in scatter radiation patterns.

Other solutions, like ceiling-mounted shielding, do not have a large floor footprint but do take away space from other ceiling-mounted equipment, such as boom-mounted lights or monitors. This increases the complexity in the hybrid OR.

### SUMMARY OF THE INVENTION

It is an object of embodiments of the present invention to provide a shielding with high flexibility, which provides good protection and which can be easily and quickly adapted so it does not pose obstruction to imaging apparatuses with low floor footprint.

This is provided by a shielding system and an operating table including such system, wherein the system can be collapsed by moving one or more shields or parts thereof in a vertical direction, for allowing movement and reposition of imaging systems (such as a C-arm) with no need to separate the shielding system away of the operating table. The vertical direction is the direction perpendicular to a plane parallel to the horizon. This is typically the length direction of a person, e.g. a medical practitioner, who is to be protected from radiation present in the operating room, and who is standing or sitting next to the operating table. For instance in embodiments where a patient support or body rest of the operating table is positioned substantially horizontally (i.e. in a plane substantially parallel to the horizon), the vertical direction is the direction substantially perpendicular to the operating table. The shielding system may be attachable or attached to an operating table.

The shielding system may include a foldable shield, such as a bottom foldable shield, for protecting the lower part of the body of medical personnel from radiation (e.g. scatter radiation), and which can be folded up to avoid contact with an imaging system. Alternatively or additionally, a vertically slidable top shield can be included, which can slide up for protecting the top part of the body of medical personnel, and which can slide down to avoid contact with an imaging system.

In a first aspect, a shielding system is provided for shielding a body from scatter radiation during a medical intervention. The shielding system comprises a frame attachable to an operating table, and a shield comprising material for blocking at least particular wavelengths of radiation, e.g. ionizing radiation such as x-rays and/or y-rays, the shield being configured for being mounted on the frame, wherein the shielding system is adapted to be collapsed by displacement of at least part of the shield in a direction substantially perpendicular to the operating table.

It is an advantage of embodiments of the present invention that protection can be obtained in a comfortable way, with an easy and fast reconfiguration which does not need to move the frame sideways or away from the operating table, thus allowing quickly changing the configuration of the shielding system when changing the position of e.g. the operating table, or a C-arm, or both, thus reducing downtime during a medical intervention. In some embodiments of the present invention, the shield comprises a bottom shield extendable from the frame downwards e.g. in the vertical direction, for protecting the lower part of the body of a medical practitioner.

It is an advantage of embodiments of the present invention that the lower part of the body, including the vulnerable genital area, can be protected from scatter radiation.

In some embodiments of the present invention, the bottom shield or at least part thereof is selectably displaceable in a folded configuration and in an unfolded configuration.

It is an advantage of embodiments of the present invention that the area of the bottom shield can be reduced and extended by folding and unfolding it in an easy and fast way.

In some embodiments of the present invention, the bottom shield is additionally or alternatively displaceable and shapeable as a staircase in a staircase configuration, for better protecting the lower part of the body of a medical practitioner in a sitting position.

It is an advantage of embodiments of the present invention that the position of the shield panels can be adapted to face the source of scattered radiation to provide protection in multiple positions, even if the practitioner is sitting down.

In some embodiments of the present invention, the bottom shield comprises displaceable panels connected to each other by rigid hinges and adapted to maintain the relative position of the panels so that the configuration of the bottom shield is kept stable until repositioned.

It is an advantage of embodiments of the present invention that the position of the shield panels can be kept with no need to contact the medical practitioner's body, thus improving comfort and scope and ease of movement of the practitioner.

In some embodiments of the present invention, the shield comprises a top shield slidable on the frame for retracting and extending the top shield in a vertical direction, for protecting the upper part of the body of a medical practitioner.

It is an advantage of embodiments of the present invention that the top part of the body of a medical practitioner (e.g. eyes) can be protected, while allowing fast reconfiguration of the shielding system when changing the position of e.g. the operating table, or a C-arm, or both, thus reducing downtime during an operation.

In some embodiments of the present invention, the top shield can be combined with a bottom shield, where a window can be left between the top shield and the bottom shield when at least the top shield is in extended configuration. The system may further comprise a set of flexible flaps with radiation shielding material for blocking the window between the top shield and the bottom shield from radiation.

It is an advantage of embodiments of the present invention that it allows temporary access of a medical practitioner to the operating table with no need of collapsing or moving a major part of the shield away, thus advantageously reducing exposure, e.g. reducing exposure area, during intervention.

In some embodiments of the present invention, the top shield comprises material for blocking at least particular wavelengths of radiation, e.g. ionizing radiation, which material is at least partially transparent to light for allowing a medical practitioner to see through the top shield.

It is an advantage of embodiments of the present invention that the top part of the body can be protected from particular wavelengths of radiation while simultaneously not blocking the field of view during operation.

In some embodiments of the present invention, the system further includes an articulated mechanism, wherein the frame is attachable to the operating table via the articulated mechanism. The mechanism allows movement of the frame and repositioning of the shield in a direction substantially parallel to the operating table.

It is an advantage of embodiments of the present invention that the shield can be moved aside for allowing wide access to the operating table, e.g. when no radiation to be blocked is present. It is an advantage that the shield can be moved aside fast in case of an emergency.

In some embodiments of the present invention, the articulated mechanism allows movement of the shield between a first side of the operating table and a second side of the operating table opposite thereto. An angle around an axis passing close to the center of the operating table can be measured from an edge of the shield in a distal position from the head of the operating table at the first side of the operating table, to an edge of the shield in a distal position from the head of the operating table at the second side of the table. In embodiments of the present invention, said angle may be larger than 180°.

It is an advantage of embodiments of the present invention that the shield can be moved around the head of the operating table, where some medical personnel (e.g. anesthesiologists) stay during interventions. It is an advantage of embodiments of the present invention that the shield can provide protection at any position around the source of scatter radiation.

In some embodiments of the present invention, the articulated mechanism comprises bars and bar hinges and pins attachable to the frame. Parts of the articulated mechanism should have sufficient strength to carry the lead panels, the lead flaps and /or the top shield. The pins may for instance be made from high-performance steel and/or titanium. The bars may be made from metal, such as steel or aluminium for instance.

It is an advantage of embodiments of the present invention that the articulated connection between the operating table and the shield can be easily provided.

In some embodiments of the present invention, the frame is slidably connectable to the articulated mechanism for allowing the shield to move in a horizontal direction, e.g. sideways with respect to the operating table.

It is an advantage of embodiments of the present invention that the positioning can be flexible, allowing a wide area of coverage and relative positioning of the shield relative to the source of radiation and the medical personnel.

In some embodiments of the present invention, the bars include at least two bars, one extreme of each bar being fixed to different axes fixed relative to the operating table around which the bars can rotate, the opposite side being connected to the frame.

It is an advantage of embodiments of the present invention that a stable and strong connection with low strain onto the articulated mechanism can be easily obtained.

In a further aspect, the present invention provides an operating table comprising the shielding system of the previous aspect of the present invention.

It is an advantage of embodiments of the present invention that an operating table can be provided which provides protection to the medical staff against radiation, and which does not hinder the intervention.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### BRIEF DESCRIPTION OF THE DRAWINGS

FIG 1 illustrates a shielding system in accordance with some embodiments of the present invention.
FIG 2 illustrates three different interchangeable configurations of a foldable bottom shield of a shielding system in accordance with some embodiments of the present invention.
FIG 3 illustrates three different interchangeable configurations of a slidable top shield of a shielding system in accordance with some embodiments of the present invention.
FIG 4 illustrates an articulated system for attaching a frame holding one or more shields to an operating table, in accordance with some embodiments of the present invention.
FIG 5 illustrates a shielding system including the articulated system for attaching the shielding system to an operating table, in accordance with some embodiments of the present invention.
FIG 6 illustrates a detail of the connection between the articulated system and the frame in a shielding system in accordance with some embodiments of the present invention.
FIG 7 illustrates a side view of an operating table with a shielding system, including a top and partially folded bottom shields, attached between the back rest and the head rest, in accordance with some embodiments of the present invention.
FIG 8 illustrates a perspective view of an operating table in accordance with some embodiments of the present invention, for two positions of the shielding system, where the articulated system allows turning the shields around the head rest of the operating table and slide along a horizontal direction, e.g. sideways with respect to the operating table.
FIG 9 illustrates the top view of FIG 8, showing two opposite configurations
FIG 10 illustrates an operating table with a collapsed shielding system and a C-arm as a radiation source for medical imaging.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### DETAILED DESCRIPTION OF ILLUSTRATIVE EMBODIMENTS

The present invention will be described with respect to particular embodiments and with reference to certain drawings but the invention is not limited thereto but only by the claims. The dimensions and the relative dimensions do not correspond to actual reductions to practice of the invention.

The terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. Thus, the scope of the expression "a device comprising means A and B" should not be interpreted as being limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

The present invention relates to a shielding system comprising a shield, which is mounted on an operating table, where an operating table comprises a patient support, which is the actual surface on which the patient lies, further called a body rest, mounted on a stand.

The body rest can, but does not need to be, placed in a horizontal position, which is a position in a plane parallel to the horizon. The shield may preferably be placed in a substantially vertical direction, the vertical direction being perpendicular to a horizontal plane. A substantially vertical direction best fits to a position a person takes when standing or sitting next to an operating table.

Different radiation shielding requirements exist for different personnel in an operating room such as a hybrid operating room. Traditional rigid shields offer high protection to some staff members, while others have lower requirements although they may also be exposed to radiation, for example in cases of emergency. Additionally, totally portable protection such as aprons do not offer protection in all circumstances. For example, from a historical and practical perspective, the anaesthesiologist often sits, on a movable stool, near the head of the patient. The lead, radiation-reducing apron, that is supposed to protect this area, lies flat on the lap of the user, leaving the genital area exposed to ionizing beams. This sitting position, near the source of radiation, can lead to tumors and cancer in the genital area.

The present invention combines the advantages of rigid, wall-like shielding with portability, without the need of wearing the shielding as in the case of aprons. The invention provides hybrid operating room staff, such as anaesthesiologists and nurses, with a dose-reducing workstation, including a protective shielding system that can be positioned in many ways around the patient. The shielding is attached to the operating table, so it guarantees at least a protective zone next to the table as it is not possible to move it far away from the operating table as in the case of wheeled shielding; however, it is easily moveable and quickly repositionable so that even in an emergency it can fulfill its purpose. The shielding system is also easily and quickly collapsible so as to allow freedom of positioning of the radiation source.

In some embodiments, despite the presence of the shields, patient access is still possible, through a vertically movable top shield. The shield may be moved up so as to open a window between top and bottom shielding and allow accessing the operating table. The window may be covered by flexible radiation-absorbing flaps. The top shielding may be transparent for instance for visible light but block scatter radiation in predetermined wavelength ranges, e.g. ionizing radiation, so as to protect medical staff.

The shielding system allows patient access. In some embodiments, the invention includes a top shield which is adjustable in height, e.g. through a rail-system, and a bottom shielding which is also adjustable in height, and/or may be foldable. When the top shielding is extended to its highest position and the bottom shielding is fully extended downwards, a human can stand around 1 meter behind the system and can be fully protected from scatter radiation. When both are at least partly collapsed, they pose no obstruction to the C-arm during change of position. In some embodiments, in case hands-on patient access is required, the top shield can be moved up, and the patient can be directly monitored through flexible radiation-absorbing shielding flaps.

To accommodate the radiation source in different types of procedures, the shield or shields can be mounted to the head rest through an articulation system, for example a four-bar mechanism, that allows the shielding system to rotate around the operation table head rest. This way, it is generally positioned perpendicular to the scatter radiation source (the patient), providing optimal protection per lead surface (e.g. per lead panel in a shield). Additionally, it can be collapsed, so as to not hinder the movement of the radiation source (such as a C-arm).

In a first aspect, the present invention provides a radiation shielding system with one or more shields for protecting at least parts of the body of medical staff from radiation, such as scatter radiation. The shield is movably attached to the operating table.

The shielding system comprises a frame and a shield which is adapted to be mounted and held by the frame. The shielding system is positioned between the medical staff and the operating table. The shielding system can be collapsed. For example, the frontal projection of the area of the shield can be moved, or for example essentially reduced. This is attained by displacement of at least parts of the shield, in a vertically upward or downward direction, e.g. in a direction which is substantially perpendicular, or at least not parallel, to a horizontal plane, e.g. the plane defined by the ground of the operating room. In embodiments of the present invention, the shield is displaceable in the direction substantially perpendicular to the operating table, for instance in the vertical direction. The skilled person would understand that even if the operating table is slightly inclined, the shield can be and collapsible so as to allow other instruments, such as a C-arm, to be moved without obstacles, e.g. without hitting the top or the bottom of the one or more shields, as they are upwardly and/or downwardly (vertically) retracted. For example, the one or more shields can be displaceable vertically with respect to the ground of the operating room (the ground being defined as horizontal, i.e. in a plane parallel to the horizon).

In some embodiments, the shielding system includes a bottom shield, extending downwards from the frame, so as to protect the lower part of the body of medical staff standing in the upright position. For example, it may expand downwards with respect to the body rest of the operating table, in the vertical direction. The bottom shield can be retracted and extended upwardly and downwardly with respect to the operating table, in the vertical direction. For example the bottom shield may be foldable, so it can be folded upwardly.

In some embodiments, the shielding system includes a top shield, slidable on the frame in the upward and downward vertical direction, for retracting and extending the top shield so as to protect the upper part of the body of medical staff.

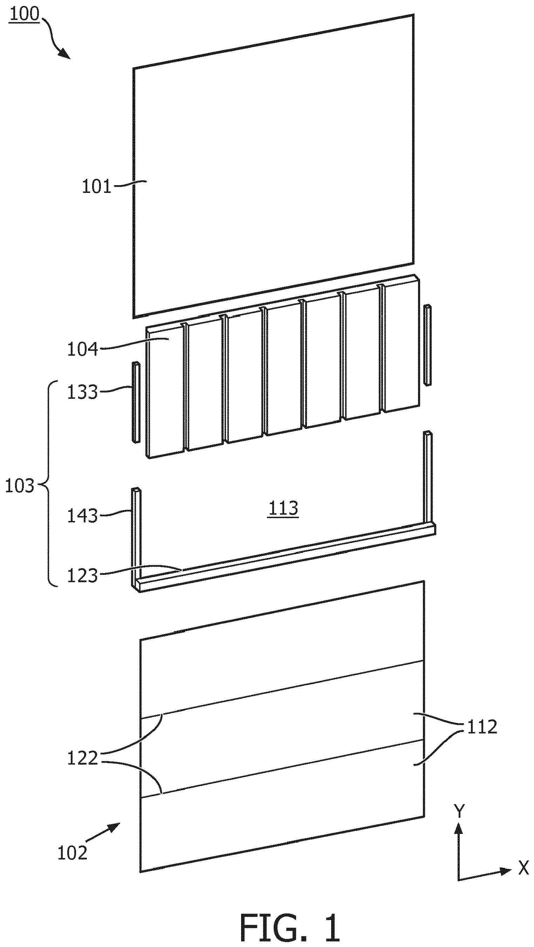

FIG 1 shows a vertically exploded perspective of an exemplary embodiment of the shielding system 100. The shield comprises at least a bottom shield 102, including at least one shielding element, e.g. at least one shielding panel 112, comprising material for shielding medical staff from radiation, for example radiation used in medical imaging, specifically for instance ionizing radiation. The bottom shield 102 is held by a frame 103. For example, the frame 103 may include a holder 123, which may be a bar or similar elongated shape, to which the bottom shield 102 can be attached or hanged.

In the embodiment shown in FIG 1, the bottom shield 102 includes three shielding elements, for example three lead panels 112 which can be folded. The present invention, however, is not limited to this number of shielding elements. The bottom shield is configured so that it allows the user to position them in a desired shape which can be conformed to the shape of the body of the medical staff in different positions. A different number of shielding elements may allow the bottom shield to accommodate to different body sizes. The bottom shield can be configured also so the shielding elements 112 can be aligned downwards to create one long shield 102, as it is shown in FIG 1. In embodiments of the present invention, the shield 102 may be positioned in a substantially vertical direction, i.e. substantially perpendicular to a plane parallel to the horizon.

Further, the embodiment of shielding system 100 of FIG 1 shows a top shield 101, which is configured to shield the upper part of the body of medical personnel from a particular wavelength range of radiation. In some embodiments the top shield 101 comprises material allowing the passing one set of wavelengths of radiation, e.g. visible radiation (light), while blocking another set of wavelengths of radiation, e.g. ionizing radiation. This way, the top shield does not block the line of sight of medical personnel, while still providing protection against particular wavelengths of radiation. In some embodiments, the top shield comprises a lead glass panel.

The top shield 101 is attached to the frame 103. The position of the top shield 101 may be adjustable vertically in upward and downward direction, so the height of the top edge of the top shield 101 can be adjusted. For example, the frame 103 may include a rail-system, comprising sliders 133 which are fixed to the top shield 101, and which can slide over slide holders 143. The rail system may include other features such as screws, tabs and the like so the height of the top shield 101 can be fixed relative to the slide holders 143, to selectively prevent or allow the top shield 101 to slide with respect to the slide holders 143.

The position of any or both of the shields can be adapted in height to form a window allowing access, e.g. by means of an arm, of medical staff to the operating table. For example, the position of the top shield 101 relative to the bottom shield 102 can be adapted, so in case hands on patient access is required, the top shield 101 can be moved so a window 113 can be left between the top and bottom shields 101, 102 for allowing reaching a patient on the operating table. The window is preferable covered by a set of flexible radiation-blocking flaps 104, for blocking a particular wavelength range of radiation. For example, the flaps may comprise a polymeric outer layer and lead, for blocking ionizing radiation. Through the flexible lead shielding flaps 104, the patient can be directly monitored.

When one or both shielding parts are at least partly collapsed, they pose no obstruction to other instruments, such as radiation sources (e.g. a C-arm) during change of position. For example, in a scenario where the C-arm is rotating around a head mount, the bottom shielding can be folded together, so they take a minimal amount of vertical space.

Hence, the shielding system provides a highly adaptable and configurable shield the shielding area of which can be easily and quickly adapted to the needs of medical staff during the different stages of a surgical operation. Not only can the shielding system be extended and retracted, but also can its shape be adapted to different configurations.

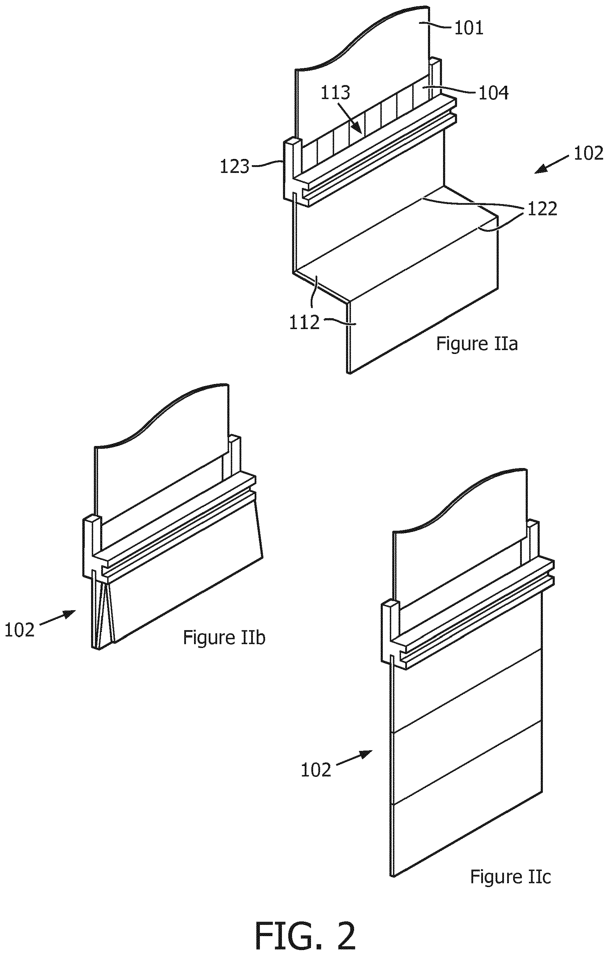

For example, in some embodiments, as shown in FIG 2 (top right drawing), the bottom shield can be shaped as a staircase. The bottom shield in a staircase configuration can provide room for knees and legs while medical staff, e.g. an anaesthesiologist, is in a sitting position. In some embodiments, the staircase configuration allows the bottom shield to extend to a region under the operating table, thus creating a space under the table that is shielded from radiation. This improves radiation protection to the abdominal and genital areas of medical staff.

For example, the foldable bottom shield 102 may comprise displaceable panels 112 which can be linked horizontally by hinges 122. These hinges 122 can keep the position of the panels 112 until repositioned by the user. For example, the hinges 122 can lock the position of the panels 112, e.g. frictionally, allowing a user to manually fold or extend the shield 102. The static friction coefficient of the hinges 122 may be high enough so as to overcome the forces by the weight of the panels 112, but low enough so that they can be moved, e.g. manually, by a user. Thus, the configuration of the bottom shield is stable until a user repositions it to a new configuration, which may also be kept stable thanks to the hinges.

The present invention is not limited thereto, and the bottom shield may include actuators so the extension, locking and folding can be electronically controlled by a user. The presence of hinges 122 is optional, and other systems for folding can be used, for example the panels could be rolled up and down.

In its folded configuration (left hand drawing in FIG 2), the bottom shield 102 is collapsed and the area of shielding is decreased. Because it occupies less space, the shielding system presents less or no obstruction to other instruments of the operating room, such as the radiation source, e.g. C-arm. In the bottom drawing, the shield 102 is fully extended, improving protection from scatter radiation to medical staff, e.g. standing nurses.

The panels 112 may comprise rigid or semiflexible lead flaps, for example opaque flaps.

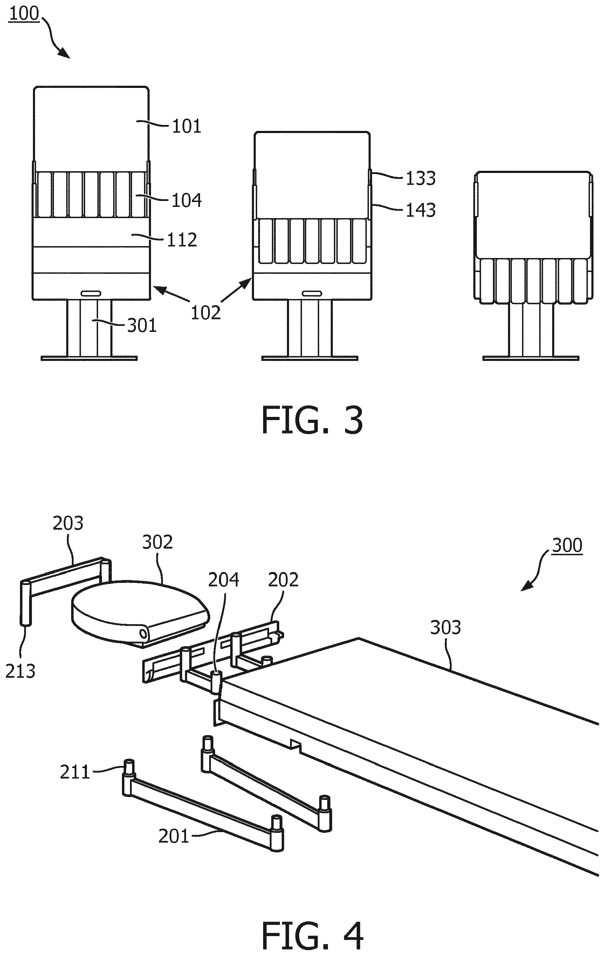

FIG 3 shows the system 100 in three stages of extension / collapse, as seen from the shielded area. The stand 301 of the operating table is shown behind the shielding system. In this case, it is shown that not only the bottom shield 102 can be collapsible so as to not disturb motion of the radiation source, but alternatively or additionally also the top shield 101 can have similar functionality. In this case, the top shield 101 is adjustable in height. A rail-system can be used, with sliders 133 attached to the top shield 101 and slide holders 143 as explained earlier, but any other system providing such relative motion can be used. As shown in FIG 3, the area of protection can be expanded for protection of medical staff, or it can be contracted to avoid hindering the motion of nearby instruments, such as a radiation source, e.g. a C-arm. Even in the expanded configuration, medical staff can reach the operating table by introducing the arm through the set of flexible radiation shielding flaps 104 covering the free area between the top shield 101 and bottom shield 102.

This is not the only option for extending, and a top shield of embodiments of the present invention may also comprise panels, e.g. transparent lead panels, which can be folded the same way as in the case of the bottom shield, with hinges, providing the same function.

The shielding system can be movably mounted to an operating table, so it is attached thereto, while being able to move it aside or along the route followed by medical staff around the operating table. For example, the shielding system may be attached to the head rest of an operating table, or to the link between the head rest and the body rest. The shielding system is attached so that the shield can rotate around the operating table, while facing it. The shielding system may present sliding motion, so the shield can slide sidewise without rotation. In other embodiments, the shielding system presents both rotation and sliding motion.

FIG 4 shows an operating table 300 in an exploded perspective view, including an articulated mechanism 200 which can be attached to or be part of the link elements between the head rest 302 and the body rest 303 of the operating table 300. The articulated system or mechanism 200 may include a mounting part or mounting bar 202 fixable to the operating table, for example fixable to the head rest 302, or to the body rest 03, or to the attachment between them or be part of said attachment. One or more articulated pieces, e.g. at least one articulation bars 201, links the shielding system to the operating table and can be attached or connected to the mounting bar. For example, the mounting bar 202 may include two attachment points which serve as rotation axes or hinges 204 for attaching pins 211 of rotatable articulation bars 201, e.g. at the extreme of the bars 201. The articulation bars 201 can rotate around the hinges 204 when attached to the mounting bar 202. The articulation bars 201 can be attached, via pins at the opposite extreme, to hinges 213 in a further, e.g. a sliding bar 203 which can in turn be connected, e.g. slidably connected, to the frame 103 holding the shields 101, 102 of the shielding system 100.

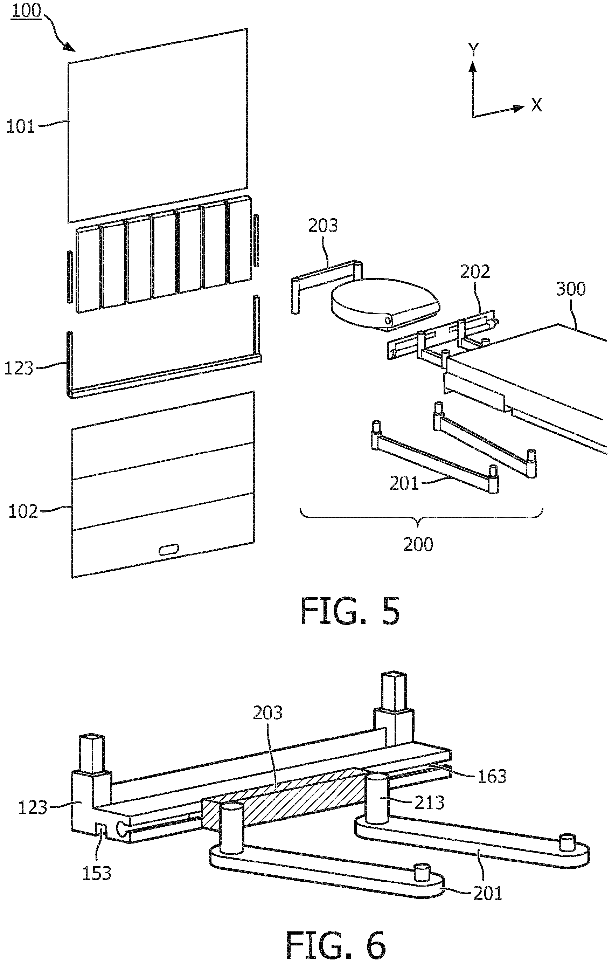

FIG 5 shows an exploded perspective view of a shielding system 100 and an operating table 300, in accordance with some embodiments of the present invention. The shielding system 100 includes two shields 101, 102 attached to a frame as in FIG 1, and it further includes the articulated mechanism 200 of FIG 4, which allows movably connecting the shields to the operating table 300. The sliding bar 203 can be connected to the frame 103 via its holder 123.

In particular, FIG 5 shows that the shields 101, 102 and frame may be connected to the operating table 300 through a four-bar mechanism including two articulating bars 201 linking the sliding bar 203 to the mounting bar 202. The hinges of the bars connect with the fitting pins of the other bars (e.g. hinges 213, 204 in the sliding and mounting bars 203, 202 with pins 211 in the articulating bars, although alternative connections can be used, such as hinges in all bars with removable pin), which allows the shielding system to rotate around the operation table head rest.

In the present embodiment, the sliding bar 203 can slide over the holder 123 of the frame 103, providing the shielding system with a further freedom of lateral positioning.

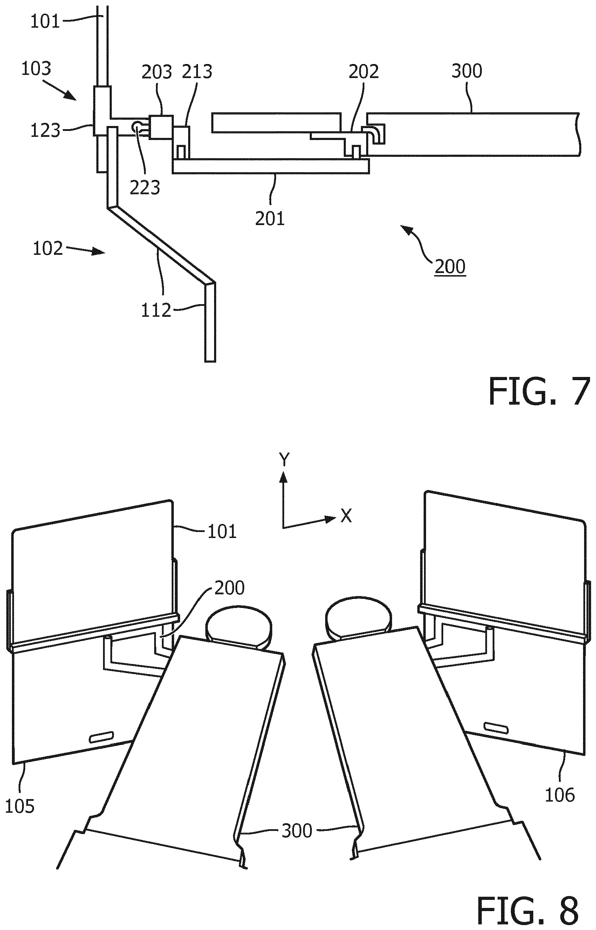

FIG 6 and FIG 7 show with greater detail the connection between the sliding bar 203 and the holder 123. The holder also shows the attaching element 153 to receive the bottom shield 102 (e.g. hooks or the like in the lower or bottom shield 102) and hold or hang it to the frame 103.

The holder 123 of the frame includes a holding element 163, e.g. a groove, matching a sliding element 223, e.g. a tab, of the sliding bar 203 of the articulated mechanism 200, to provide the frame 103 and the attached shield or shields 101, 102 with sidewise motion relative to the operating table 300 or to the ground of the operating room, e.g. in the horizontal direction (X). The sliding element 203 connects to the articulating arms 201 via a hinge-pin connection, as mentioned with reference to FIG 4. This is a simple and reliable type of connection which allows a combination of rotation and sliding. Of course, the present invention is not limited to a groove and sliding tab in the holder and connector, and any other equivalent sliding elements can be used as long as they provide sidewise motion of the shields in a direction following the horizontal plane. For example, a railing system can be used, and/or a single arm may be connected to the frame via a slidable hinge rather than to a sliding bar with two hinges, or a hinge with a groove for receiving a protrusion on the frame, allowing sliding motion.

FIG 7 shows a cross section of (part of) the operating table 300 attached to the shielding system. The articulation mechanism 200 links the shields 101, 102 and frame 103 to the operating table. The mounting bar fixed to the operating table 300 is linked to the sliding bar 203 via the articulating bars 201. The sliding bar is connected to the holder 123 of the frame 103 via the sliding system provided by the groove and fitting tab 223 inserted thereto.

The lower or bottom shield 102 is shown as partially bent, allowing protection of the lower body, including genital area, of medical personnel, even in the sitting position next to the head rest 302 of the operating table.

Because of the combination of rotation and slide in the embodiments of the present invention shown in the figures, the positioning of the shields can be flexible, allowing a wide area of coverage and relative positioning of the shield relative to the source of radiation and the medical personnel.

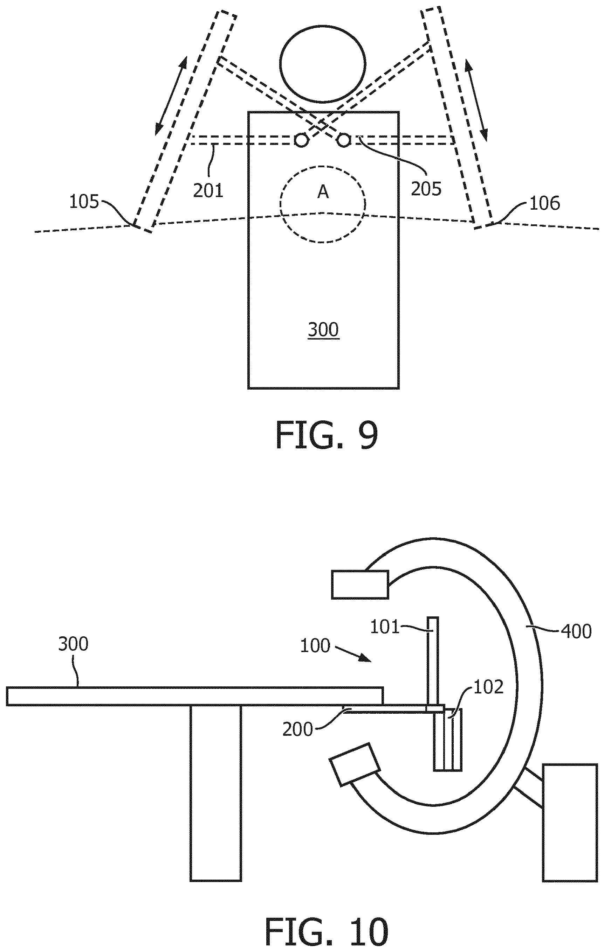

FIG 8 and FIG 9 show positioning of the shielding system to protect different regions from radiation scattered from the area containing the operating table. FIG 8 shows two positions of the shields with respect to the operating table. The region next to the head rest can be shielded from radiation interchangeably at one or the other side of the operating table. Sliding the shields with respect to the articulated bars and rotating the articulating bars allow an easy positioning of the shields of the system with respect to the operating table with great freedom. For example, FIG 9 shows two different positions of the shields, where a first and second articulating bars 201 are allowed to rotate around a first and second axis 205, which may be determined by the position of the hinges 204 of the mounting bar (not shown in FIG 8 and FIG 9).

In some embodiments of the present invention, the shielding system may transition between two opposite extreme positions, between a first side of the operating table 300 and the opposite side. An angle A can be defined around an axis passing at or close to the center of the operating table, and perpendicular thereto, the angle being measured between two edges of the shield, in particular from an edge 105 of the shield in a distal position from the head of the operating table at the first side of the operating table 300, to a second edge 106 of the shield in a distal position from the head of the operating table 300 at the second side thereof. In some embodiments, the angle A is larger than 180°. For example, the rotation of the articulating arms is done around different axis, so their angle of rotation is limited, and one of the bars can be stopped by the other bar, so the position of the edge 105, 106 may be limited by the relative rotation of the bars 201. Thanks to the sliding mechanism, the area of the shield can be repositioned while the bars are fixed by sliding the sliding bar over the frame. Thus, the angle A can be increased by sliding the edges. In the embodiment shown in FIG 9, the angle A may be around 190°, for example. This way, the shields can be generally positioned perpendicular to the scatter radiation source (e.g. the radiation source, and/or the patient), providing optimal protection per lead surface.

However, the invention is not limited to such configuration. For example, the articulation mechanism 200 may allow only sliding of the shields, e.g. sliding with respect to the operating table, e.g. around it. In other embodiments, the articulation system may allow rotation of the shields around the operating table but not sliding, for example by attaching without allowing sliding the at least one articulating bar 201 to the shields or the frame holding them.

In another aspect, an operating table including the shield system of the first aspect is provided. The operating table includes a frame and at least one shield. The frame holding the at least one shield is connected to the operating table by an articulation system, as explained with reference to embodiments of the first aspect. Thus, an operating table is provided with high safety and radiation shielding for medical personnel. The shields can rotate around the operating table, e.g. the head rest thereof, allowing complete freedom of movement to medical staff with no need to separate and set aside the system from the operating table. The shielding system is also collapsible, so that it does not disturb other instruments of the operating room, such as the radiation source (e.g. C-arm). FIG 10 shows an operating table 300 with a shielding system 100, including a top and bottom shields 101, 102 attached to the table 300 by an articulation system. The top shield 101 is retracted and the bottom shield 102 is in a folded configuration, so the C-arm 400 can be moved around the headrest of the operating table 300 without being disturbed by the shielding system 100.

As explained earlier, the shielding system also allows reaching the patient on the table while at the same time protecting the medical staff, by allowing to pull up the top shield so a window covered in flexible lead flaps (or similar radiation shielding flexible curtains) is opened and the patient can be reached with no need to set the shield aside to reach the patient.

## Claims

1. A shielding system (100) for shielding a body of a user from scatter radiation during a medical intervention, the shielding system (100) comprising:
a frame (103) attachable to an operating table (300), and
a shield (101, 102) comprising material for blocking at least particular wavelengths of radiation, the shield (101, 102) being configured for being mounted on the frame (103),
wherein the shielding system (100) is adapted to be collapsed by displacement of at least part of the shield (101, 102) in a vertical direction (Y).

2. The system in accordance with claim 1, wherein the shield comprises a bottom shield (102) extendable from the frame (103) downwards in the vertical direction (Y), for protecting the lower part of the body of the user.

3. The system (100) in accordance with claim 2, wherein the bottom shield (102) or at least part thereof is selectably displaceable from a folded configuration to an unfolded configuration, and vice versa.

4. The system (100) in accordance with any of claims 2 or 3, wherein the bottom shield (102) is displaceable and shapeable as a staircase in a staircase configuration, for protecting the lower part of the body of the user in a sitting position.

5. The system (100) in accordance with any one of claims 2 to 4, wherein the bottom shield (102) comprises displaceable panels (112) connected to each other by rigid hinges (122) and adapted to maintain the relative position of the panels (112) so that the configuration of the bottom shield (102) is kept stable until repositioned.

6. The system (100) in accordance with any of the previous claims, wherein the shield comprises a top shield (101) slidable on the frame (103) for retracting and extending the top shield (101) in a vertical direction (Y), for protecting the upper part of the body of the user.

7. The system (100) in accordance with any one of claims 2 to 5, wherein the shield further comprises a top shield (101) slidable on the frame (103) for retracting and extending the top shield (101) in a vertical direction (Y), for protecting the upper part of the body of the user, where a window (113) is left between the top shield (101) and the bottom shield (102) when at least the top shield (101) is in extended configuration, and further comprising a set of flexible flaps (104) comprising radiation shielding material for blocking the window (113) between the top shield (101) and the bottom shield (102).
T

8. The system (100) in accordance with any of claim 6 or 7, wherein the top shield (101) comprises material for blocking at least particular wavelengths of radiation, and which is at least partially transparent to light for allowing a user to see through the top shield (101).

9. The system in accordance with any one of the previous claims, further including an articulated mechanism (200), wherein the frame (103) is attachable to the operating table (300) via the articulated mechanism (200) for allowing movement of the frame (103) and repositioning of the shield (101, 102) in a substantially horizontal direction (X).

10. The system in accordance with claim 9, wherein the articulated mechanism (200) allows movement of the shield (101, 102) between a first side of the operating table (300) and a second side of the operating table (300) opposite thereto, where an angle (A) around an axis passing through the center of the operating table measured from
an edge (105) of the shield (101, 102) in a distal position from the head of the operating table at the first side of the operating table (300), to
an edge (106) of the shield in a distal position from the head of the operating table at the second side of the table (300)
is larger than 180°.

11. The system (100) in accordance with any of claims 9 or 10, wherein the articulated mechanism (200) comprises bars (201, 202, 203) and bar hinges and pins (204, 213) attachable to the frame.

12. The system (100) in accordance with any of claims 9 to 11, wherein the frame (103) is slidably connectable to the articulated mechanism (200) for allowing the shield (101, 102) to move in a substantially horizontal direction (X).

13. The system (100) in accordance with any one of the claims 11 or 12, wherein the bars include at least two bars (201), one extreme of each bar being fixed to different axes (205) fixed relative to the operating table (300) around which the bars (201) can rotate, the opposite side being connected to the frame (103).

14. An operating table (300) comprising the shielding system (100) in accordance with any one of claims 1 to 13.
